# EUROPEAN PATENT SPECIFICATION

(11) **EP 3 675 833 B1**
(45) Date of publication and mention of the grant of the patent: **10.06.2026**
(21) Application number: 18768790.0
(22) Date of filing: 29.08.2018
(51) Int. Cl.: A61K 9/48

(54) **STABLE CAPSULES WITH FECAL MICROBIOTA OR A CULTURE OF MICROORGANISMS**
STABILE KAPSELN MIT FÄKALIENMIKROBIATA ODER EINER KULTUR VON MIKROORGANISMEN
CAPSULES STABLES AVEC MICROBIOTE FÉCAL OU CULTURE DE MICRO-ORGANISMES

(30) Priority: 29.08.2017 EP 17188258
(43) Date of publication of application: 08.07.2020
(73) Proprietor: Chr. Hansen A/S, 2970 Hoersholm (DK)
(72) Inventor: YDE, Birgitte, 2970 Hoersholm (DK); GÜNTHER, Stig, 2640 Hedehusene (DK)
(74) Representative: NVS EPO Representatives
(86) International application number: PCT/EP2018/073217
(87) International publication number: WO 2019/043051

(56) References cited:
- WO-A1-2016/065279
- WO-A1-2016/178775
- GB-A- 1 190 386
- US-A- 5 310 555
- US-A1- 2012 039 998
- US-A1- 2017 189 363

## Description

### FIELD OF THE INVENTION

The present invention relates to a hard capsule comprising a hard outer and one inner hard capsule, wherein the outer capsule comprises an edible oil and the inner hard capsule which comprises a composition comprising microorganisms which are not coated or microencapsulated and at least one desiccant, wherein the hard capsule has a load of microorganisms of at least 30% weight/volume.

### BACKGROUND OF THE INVENTION

Fecal microbiota transplantation (FMT) is the transfer of fecal material containing microorganisms from a healthy individual into a diseased recipient. Previous terms for the procedure include fecal bacteriotherapy, fecal transfusion, fecal transplant, stool transplant, fecal enema, and human probiotic infusion (HPI). Because the procedure involves the complete restoration of the entire fecal microbiota, not just a single agent or a combination of agents, these terms have now been replaced by the new term: "fecal microbiota transplantation".

Traditionally, transplantation to the upper GI tract is achieved via naso-gastric, naso-duodenal, naso-jejunal intubation, or via esophagogastroduodenoscopy or push enteroscopy. Delivery to the lower GI tract is usually achieved by colonoscopy, sigmoidoscopy, or enema. All of these techniques suffer from shortcomings. For example, upper GI tract administration carries the risks of aspiration-related complications (particularly naso-gastric delivery) and is invasive and uncomfortable to recipients. Lower GI tract delivery techniques such as colonoscopy and sigmoidoscopy are also invasive and uncomfortable and are associated with significant costs and risks.

Accordingly, there remains a need for a safe, effective, and less invasive manner for delivery of microbial communities to recipients (e.g., fecal matter transplant or fecal microbiota transplantation). Compositions and methods that meet these objectives would be critical for improving the treatment of gastrointestinal dysbiosis, including treatment of recurrent *Clostridium difficile* infection as well as dysbiosis associated with various chronic diseases.

Two approaches have been pursued for developing encapsulated oral formulations of microbial communities. The first approach involves flash-freezing of an aqueous solution of stool in a glycerol and saline buffer. The aqueous solution preserves the viability of the microbial strains but produces capsules that are highly unstable as the aqueous character of the stool quickly degrades the water-soluble capsules. The physical instability of these capsules complicates mass-production and creates clinical hazards as the capsules can rupture during administration. The second approach involves dewatering of the microbial community through techniques such as lyophilization. However, the dewatering process is physically demanding and reduces the viability of the microbes significantly.

FMT has proven to be useful for treatment of infection with *Clostridium difficile.* In May 1988, the Centre for Digestive Diseases (CCD) in Sydney, Australia, treated the first idiopathic colitis patient with FMT which resulted in a durable clinical and histological cure. Since that time, a number of publications have reported the successful treatment of ulcerative colitis with FMT, with clinical trials now underway in this indication. Other studies are in progress to investigate if feces transplantation can be used for treatment of other diseases e.g. obesity, diabetes II, IBS, and mental diseases.

In 2012, a team of researchers from the Massachusetts Institute of Technology founded OpenBiome, the first public stool bank in the United States. OpenBiome provides clinicians with frozen, ready-to-administer stool samples for use in treating *C*. *difficile* and supports clinical research into the use of fecal transfer for other indications.

Researchers have also produced a standardized filtrate composed of viable fecal bacteria in a colourless, odourless form. The preparation has been shown to be as effective at restoring missing and deficient bacterial constituents as crude homogenised FMT.

WO2014121298 describes the production of capsules with fecal matter suspended in glycerol and filled into capsules which were immediately capped and placed onto an aluminium freezing block held at -80°C via dry ice to freeze. The frozen capsules were in turn over-capsulated to enhance capsule stability and placed into <-65°C storage immediately. The final product was stored at <-65°C until the day and time of use. On the day of dosing, capsules were warmed on wet ice for 1 to 2 hours to improve tolerability and were then dosed with water ad libitum.

WO2016/178775 describes compositions and methods for therapeutic delivery of microbial communities. The composition comprises an aqueous sample of a microbial community and an emulsifying agent encapsulated within a water-soluble capsule, wherein the aqueous sample and the emulsifying agent form a water-in-oil emulsion, and the pharmaceutical is stable at room temperature for at least about 30 minutes.

WO2016/065479 describes a therapeutic capsule for the oral administration of bacteria to the gastrointestinal system, comprising a capsule shell enveloping a lipophilic matrix permeated with discrete microcapsules, wherein each microcapsule is a lipophilic matrix comprising an aqueous medium, stabilized into a discrete structure by a colloidal polymer, and containing the bacteria. The microparticles have a particle size about 0.1 micrometers to 3,000 micrometers. A capsule may have about 5% to about 20% weight/volume of live bacteria.

US 2012/0039998 describes a process of manufacturing a soft gel capsule containing microencapsulated probiotic bacteria which have been coated with at least one vegetable lipid having a melting point of between 35°C and 75°C.

GB 1 190 386 describes a lactic acid bacteria drug resistant to antibiotics which comprises an enteric-coated gelatin capsule containing living lactic acid bacteria absorbed in sterilised starch.

US 5,310,555 discloses a dietary adjunct for livestock which includes incompatible live microbial cultures, and vitamin and mineral supplements, each separated from the other via multiple encapsulation.

US 2017/189363 discloses an oral pharmaceutical composition for oral administration of a therapeutic protein or peptide, comprising (a) a gastric acid modulator in an amount effective to decrease gastric acids levels in the stomach, and (b) a therapeutically effective amount of a protein or peptide which may be provided in a capsule-in-capsule dosage form, i.e. wherein the protein or peptide is contained in an inner capsule and the gastric acid modulator is contained in an outer capsule. The aim is to increase the oral bioavailability of the protein or peptide.

### SUMMARY OF THE INVENTION

If fecal microbiota transplantation could be provided using capsules having an appropriate stability, the treatment could be done by the recipient at home, avoiding transportation to a medical clinic. Accordingly, the problem to be solved is to provide a capsule of fecal microbiota with sufficient stability to avoid leakage prior to the consumption by the recipient and which can be stored at a conventional freezer at -18°C rather than at -65°C or at -80°C meaning that the capsules can be stored in a conventional freezer in the home of the recipient.

The present inventors have surprisingly discovered that by using a hard capsule having an outer and one inner capsule comprising the fecal microbiota and adding a desiccant to the composition comprising microorganisms and/or a hydrophobic liquid between the inner and outer capsule, the stability of the capsule system is increased dramatically, and the capsule can be stored at -18°C for months without being sticky or leaking. As evident to a person of ordinary skill in the art, it is a significant advantage to avoid the procedure of freezing the fecal microbiota to -65°C or at -80°C as this low temperature will inevitably kill a number of species of microorganisms present in the microbiota which might have been useful for the recipient. The hard capsules of the present invention have the advantage that the recipient can be provided with a sufficient amount of capsules to be taken for months thus avoiding numerous visits for the recipient at a medical clinic and the advantage for the medical clinic that there is no need to assign staff to prepare for and provide capsules to the recipient at the numerous visits.

### DETAILED DISCLOSURE OF THE INVENTION

The present invention relates to a hard capsule comprising a hard outer and one inner hard capsule, wherein the outer capsule comprises an edible oil and the inner hard capsule which comprises a composition comprising microorganisms which are not coated or microencapsulated and at least one desiccant, wherein the hard capsule has a load of microorganisms of at least 30% weight/volume.

US2012/0039998 describes "microencapsulated" probiotic bacteria and that the term microencapsulated means coated with a composition. The term "microcapsule" used in WO2016/065279 is defined in a similar manner. Consistent herewith, the term "uncoated" used in the present specification and claims means that the probiotic bacteria are not coated or microencapsulated to form microcapsules.

A microencapsulation or coating step may well lead to a cell loss during the microencapsulation or coating process and will also lead to a lower concentration of microorganisms through the addition of coating material to the formulation.

A major advantage of the present invention is that the hard capsule of the invention can have a load of microorganisms which is much higher than the maximum of 20% weight/volume contemplated in WO2016/065479, such at least 25%, at least 30%, at least 35%, at least 40%, at least 45%, at least 50%, at least 55%, at least 60%, at least 65%, at least 70%, at least 75%, or about 80%, such as about 77% to 82%, the actual load of microorganisms depending on the concentration of microorganisms in the fill material and the size of the internal and external capsules.

Another advantage of the present invention compared to the prior art is that there are fewer process steps which particularly for anaerobic bacteria minimizes the risk of loss of viable bacteria.

A further advantage compared to US2012/0039998 is that the production of the capsules of the invention does not require drying which can lead to a significant loss of viable bacteria.

The present invention provides a hard capsule comprising an outer and one inner capsule, wherein the outer capsule comprises a hydrophobic liquid and the inner capsule which comprises a composition comprising uncoated microorganisms and at least one desiccant.

As used herein the term "capsule" refers to a conventional hard capsule intended for oral administration to a human or animal being. The capsules of the present invention do not structurally depart from the conventional definition of hard capsules. When reference is made herein to "capsule" it refers to the outer or inner capsule or the outer capsule comprising the inner capsule unless the context indicates otherwise. Generally, the term "capsule" refers to both empty and filled capsules whereas "shell" specifically refers to an empty capsule.

As known to the person of ordinary skill in the art, commercially available capsules provided as ordinary capsules or elongated capsules are named by numbers and the suffix el for elongated capsules and have the following dimensions:

| Size | 000 | 00 | 0 | 1 | 2 | 3 | 4 | 5 |
|---|---|---|---|---|---|---|---|---|
| Volume (ml) | 1.37 | 0.91 | 0.68 | 0.50 | 0.37 | 0.30 | 0.21 | 0.13 |
| Length of closed capsule (mm) | 26.1 | 23.3 | 21.7 | 19.4 | 18.0 | 15.9 | 14.3 | 11.1 |
| External diameter Body (mm) | 9.55 | 8.18 | 7.34 | 6.63 | 6.07 | 5.57 | 5.05 | 4.68 |
| External diameter Cap (mm) | 9.91 | 8.53 | 7.64 | 6.91 | 6.35 | 5.82 | 5.32 | 4.91 |

| Size | 00el | 0el | 1el | 2el | 4el |
|---|---|---|---|---|---|
| Volume (ml) | 1.02 | 0.78 | 0.54 | 0.41 | 0.21 |
| Length of closed capsule (mm) | 25.3 | 23.1/24.2 | 20.42 | 19.3 | 15.8 |
| External diameter Body (mm) | 8.18 | 7.34/7.36 | 6.63 | 6.09 | 5.05 |
| External diameter Cap (mm) | 8.53 | 7.65/7.66 | 6.91 | 6.36 | 5.31 |

To be useful for the present invention the outer capsule should generally be slightly larger than the inner capsule. In the examples, a combination of outer capsule 00 and inner capsule 0 is used. Evidently, other combinations may be used for the present invention, e.g. as follows:

| Outer capsule | Inner capsule |
|---|---|
| 000 | 00, 00el, 0, 0el, 1, 1el, 2, 2el, 3, 4, 4el, or 5 |
| 00 | 0, 1, 1el, 2, 2el, 3, 4, 4el, or 5 |
| 0 | 1, 1el, 2, 2el, 3, 4, 4el, or 5 |
| 00el | 0, 0el, 1, 1el, 2, 2el, 3, 4, 4el, or 5 |
| 0el | 1, 1el, 2, 2el, 3, 4, 4el, or 5 |

Examples of capsule material are gelatine, polyvinyl alcohol, starch, and starch derivatives, such as hydroxypropyl starch, and starch derivatives other than hydroxypropyl starch, pectin, cellulose, celluloses derivatives such as hydroxypropylmethylcellulose (HPMC), and cellulose derivatives other than HPMC, and mixtures thereof e.g. special formulated acid-resistant HPMC resulting in acid resistant capsules. The capsules of the present invention may also be made of bacterial or yeast-derived film-forming polymers (exo-polysaccharides) such as the pullulan. Other typical exo-polysaccharides are xanthan, acetan, gellan, welan, rhamsan, furcelleran, succinoglycan, scleroglycan, schizophyllan, tamarind gum, curdlan, dextran, and mixtures thereof. If desired, the capsule may be enteric coated or prepared to provide a delayed release.

In order to fill a capsule, a solid dispersion or solution of fill components is prepared and filled into the shell. In some instances, the water soluble fill components migrate into the shell causing it to disintegrate, deteriorate or penetrate, the capsule shell. Water-soluble materials having a high degree of dissociation such as a composition of microorganisms as disclosed herein is an example of such destructive fill.

The present invention provides the solution that before or shortly after the inner capsule shell is filled with a composition comprising microorganisms it is placed in an outer capsule filled with a hydrophobic liquid in order to avoid leakage of composition and to reduce oxygen exposure of the contents.

The term "hydrophobic" refers to the property of a substance to repel water. Examples of hydrophobic substances are oils, fats, waxes, alkanes, and most other organic substances.

In the present context, the hydrophobic substance is to be liquid at a temperature above 5°C. Examples of presently preferred hydrophobic liquids are edible oils such as sunflower oil, olive oil, rapeseed oil, maize oil, soya oil, linseed oil, groundnut oil, sesame oil, rice oil, thistle oil, grape seed oil, peanut oil, coconut oil, and fish oil.

In a presently preferred embodiment, the edible oil comprises at least one antioxidant. Examples of antioxidants to be used as antioxidant in the edible oil of the present invention are ascorbyl palmitate (E304), tocopherol- rich extract (E306), alpha-tocopherol (E307), gamma-tocopherol (E308), delta-tocopherol (E309), propyl gallate (E310), octyl gallate (E311), dodecyl gallate (E312), β-carotenoids, tertiary-butyl hydroxyquinone (TBHA) (E319), butylated hydroxyanisole (BHA) (E320), butylated hydroxytolvene (BHT) (E321), lecithin (E322), 4-hexylresorcinol (E586), and combinations thereof.

The composition comprising at least one microorganism such as a bacterium, archaeae, fungus such as yeast, or virus may comprise at least one desiccant. A conventionally used desiccant which can be used for preparing a solid dispersion of the fill material is silicate, such as calcium silicate or aluminium silicate. In a presently preferred embodiment, however, the desiccant is a food approved component which is degradable in the gastrointestinal tract. Examples of such desiccants are starch, such as potato starch, corn starch, rice starch, wheat starch, or cassava starch.

The composition may also comprise at least one cryoprotectant. Examples of cryo-protectants which can be used are glycerol, carbohydrate, water soluble antioxidants such as sodium ascorbate, glutathione, riboflavin, L-cysteine, and salts or combinations thereof.

In one aspect of the invention, the capsule according to the invention comprises a composition comprising a fecal microbiota.

In the present context, the term "microbiota" refers to the community of microorganisms that occur (sustainably or transiently) in and on an animal subject, typically a mammal such as a human, including eukaryotes, archaea, bacteria, fungi such as yeasts, and viruses (including bacterial viruses i.e., phage).

The composition comprising a fecal microbiota can be prepared by a process comprising the steps of: (a) providing a fecal material obtained from a suitable donor; and (b) subjecting the fecal material to at least one processing step under conditions such that a homogenized composition of bacteria, archaea, fungi, and vira, is produced from the fecal material.

The fecal material should preferably be protected from oxygen e.g. by covering the sample immediately after producing it with oxygen reduced saline solution and by doing most of the processing in an anaerobic environment either by using an anaerobic chamber or by flushing with e.g. Ar, N₂ or CO₂.

In a presently preferred embodiment, feces and saline is homogenized, filtered and centrifuged. The supernatant is discarded, and the pellet mixed with glycerol as a cryo-protectant to provide feces extract.

The capsule according to the invention comprises a composition comprising a fecal microbiota will comprise an unknown but large number of types of microorganisms.

In another aspect, the invention provides a capsule, wherein the composition comprises a limited number of different types of microorganisms, e.g. bacteria, achaeae, fungi such as yeast, or vira including bacteriophages.

As used herein, a "type" or more than one "types" of bacteria, archeae, fungi such as yeast, or vira may be differentiated at the genus level, the species level, the sub-species level, the strain level or by any other taxonomic method, as described herein and otherwise known in the art.

In a presently preferred embodiment, the composition comprises at least one type of microorganisms which has been cultured under conditions which are appropriate for the microorganism in question, and purified.

As used herein, a microorganism is "pure" if it is substantially free of other components. The terms "purify", "purifying", and "purified" refer to a microorganism such as a bacterium or other material that has been separated from at least some of the components with which it was associated either when initially produced or generated (e.g., whether in nature or in an experimental setting), or during any time after its initial production. A bacterium or a bacterial population may be considered purified if it is isolated at or after production, such as from a material or environment containing the bacterium or bacterial population, or by passage through culture, and a purified bacterium or bacterial population may contain other materials up to about 10%, about 20%, about 30%, about 40%, about 50%, about 60%, about 70%, about 80%, about 90%, or above about 90% and still be considered "purified".

Presently preferred microorganisms to be cultured are bacteria and yeast. Yeasts are eukaryotic, single-celled microorganisms classified as members of the fungus kingdom. They are estimated to constitute 1% of all described fungal species. An example of a yeast which is considered to be useful in the capsule of the invention is *Saccharomyces boulardii.*

In a presently preferred embodiment, the microorganism is a bacterium or archeae isolated from mammalian-associated biome such as human biome. Examples of bacteria and archaea to be included in a composition to be filled into the inner capsule of the invention are members of phyla associated with human and animal microbiomes, such as Bacteroidetes (e.g., *Bacteroides* sp., such as *Bacteroides thetaiotaomicron, Prevotella* sp., etc.), Firmicutes (e.g., *Anaerostipes caccae, Blautia producta, Dorea longicatena, Eubacterium* sp., such as *Eubacterium hallii* etc., *Enterococcus faecium, Faecalibacterium* sp., such as *Faecalibacterium prausnitzii, Lactobacillus, Roseburia inulinivorans),* Actinobacteria (e.g., *Propionibacterium* sp., such as *Propionibacterium acnes, Bifidobacterium* sp., such as *Bifidobacterium bifidum, Bifidobacterium breve, Bifidobacterium infantis,* etc.), Veruccomicrobia (e.g., *Akkermansia* sp. such as *Akkermansia muciniphila*), and Proteobacteria (e.g., *Escherichia coli*)*.* Examples of archaea to be included in a composition to be filled into the inner capsule of the invention are members of phyla associated with human and animal microbiomes, such as Euryarchaeota (e.g., *Methanosphaera* sp., *Methanobrevibacter* sp., *Methanomassiliicoccus* sp., etc.).

Combinations of several species or strains of microorganisms can be included in the composition, i.e. 2, 3, 4, 5, 6, 7, 8, 9, 10, 11, 12, 13, 14, 15, 16, 17, 18, 19, 20, 21, 22, 23, 24, 25 or even more. In a presently preferred embodiment no more than 20 different types of microorganisms, e.g. bacteria, archaeae, fungi such as yeast, or vira are present in the capsule. In some embodiments only one, two, three, four or five different strains of the above listed species and strains are present in the capsule of the invention.

When it is desired to provide the recipient with a number of different types of microorganisms, this can be done by combining cultures of the various microorganisms into a combined culture to be filled into one capsule.

Alternatively, it can be done by preparing a capsule comprising a composition for each microorganism and the combination is done by the recipient by taking the desired number of capsules with the desired species of microorganisms.

The capsule of the invention is useful for populating the gastrointestinal tract of any subject such as a human recipient by oral administration to the subject of an effective amount of a composition comprising microorganisms. Depending on the severity and present status of the disease, disorder or condition the recipient may be considered a patient and the term "subject in need thereof" includes both. Unless the context indicates otherwise, all three terms are meant to designate the human or animal ingesting one or more of the capsules of the invention.

The term "subject" as used herein refers to any mammal, including, but not limited to, livestock and other farm animals (such as cattle, goats, sheep, horses, pigs and chickens), performance animals (such as racehorses), companion animals (such as cats and dogs), laboratory test animals and humans. Typically, the subject is a human.

The capsules comprising the composition may treat, prevent, delay or reduce the symptoms of diseases, disorders and conditions associated with a dysbiosis. More specifically, the capsules of the present invention may be useful for preventing or treating an infection caused by C. *difficile, Salmonella* spp., enteropathogenic *E coli,* multi-drug resistant bacteria such as *Klebsiella,* and *E. coli,* Carbapenem-resistent *Enterobacteriaceae* (CRE), extended spectrum beta-lactam resistant *Enterococci* (ESBL), and vancomycin-resistant *Enterococci* (VRE).

The present invention provides methods of treating or preventing a disease, disorder or condition associated with a dysbiosis, comprising administering an effective amount of the capsules described herein to a subject in need thereof. Representative diseases, disorders and conditions potentially associated with a dysbiosis include, but are not limited to, those mentioned below.

In some embodiments, the subject has inflammatory bowel diseases (IBD), for example, Crohn's disease, colitis (e.g., ulcerative colitis or microscopic colitis), or pouchitis; or has irritable bowel syndrome or functional dyspepsia. In some embodiments, the subject has hepatic disease, such as non-alcoholic steatohepatitis (NASH), non-alcoholic fatty liver disease (NAFLD), hepatic encephalopathy, primary sclerosing cholangitis (PSC), autoimmune hepatitis, or drug-induced liver injury. In some embodiments, the subject has an autoimmune disease such as celiac disease or eosinophilic esophagitis. In some embodiments, the subject has a hyperproliferative disease or malignancy of the GI, such as colorectal cancer/polyps, esophageal cancer or Barett's esophagus. In some embodiments, the subject has metabolic disease, such as metabolic syndrome, Type 1 or Type 2 diabetes, obesity, malnutrition or undernutrition, or cardiovascular disease (e.g., atherosclerosis). In other embodiments, the subject has rheumatologic disease, such as inflammatory arthritis (rheumatoid arthritis or RA, ankylosing spondylitis, psoriatic arthritis, IBD spondyloarthropathy), fibromyalgia, chronic fatigue syndrome, or an autoimmune and connective tissue disorder (e.g., systemic lupus erythematosus, scleroderma, and Sjogren's syndrome). In some embodiments, the subject has vasculitis (e.g., polymyalgia rheumatic/giant cell arteritis or polyarteritis nodosa). In some embodiments, the subject has a psychiatric disorder such as mood disorder (e.g., depression or bipolar disorder), anxiety disorder (e.g., general anxiety disorder, post-traumatic stress disorder), or developmental disorder (e.g., autism spectrum disorder, attention deficit hyperactivity disorder). In some embodiments, the subject has one or more of colonic polyps, cysts, diverticular disease, constipation, intestinal obstruction, malabsorption syndrome, ulceration of the mucosa, and diarrhoea. Other examples of diseases or disorders which may be treated with the capsule of the invention are atopic dermatitis, rhinitis and upper respiratory tract infection (URTI). Additional diseases, disorders and conditions which are suitable for treatment with the compositions and methods of the invention are described in Table 3 of WO 2014/121298.

As the microbiome of subjects with chronic disease or disorder tends to revert back to one's own intrinsic abnormal microbiome, repeated administration of microbial communities may be needed to ensure a sustained clinical cure. Accordingly, the capsules of the invention comprising microorganisms may be delivered as maintenance doses. The maintenance dosing regimen may vary, including by microbial dose, frequency of administration, administration interval and length, and depending on the disease and biology of the subject.

For example, therapy of chronic medical disease may require a dose of about 5 to about 50 capsules for induction therapy, such as about 5 to about 40 capsules per administration. For example, the composition may be administered at a dose of about 10, about 15, about 20, about 25, about 30, about 35, about 40, about 45, or about 50 capsules per administration. A subject may be treated one or more times. For maintenance therapy, capsules may be administered daily, or from two to five times weekly, or from one to ten times monthly. Maintenance therapy may proceed for several weeks to several months. For example, maintenance therapy may proceed for about two to about six weeks (e.g., about one month), or may proceed for about two to about six months (e.g., from about two to four months) or even longer. An "administration" refers to the capsules ingested over the course of a single day.

### LEGEND TO FIGURES

Photographs of capsules after storage at -18°C
Figure 1 shows the results of trial 1: No oil - no desiccant - 7 days of storage at -18°C.
Figure 2 shows the results of trial 2: Glycerol - no desiccant - 10 days of storage at -18°C.
Figure 3 shows the results of trial 3: Olive oil - 2.5 months at -18°C.
Figure 4 shows the results of trial 4: Rapeseed oil - 2.5 months at -18°C.
Figure 5 shows the results of trial 6: 2% calcium silicate - no oil - 1 month at -18°C.
Figure 6 shows the results of trial 7: 2% calcium silicate - rapeseed oil - 3 months at -18°C.
Figure 7 shows the results of trial 8: 10% potato starch - olive oil - 3 months at -18°C.
Figure 8 shows the results of trial 9: 10% maize starch - olive oil - 3 months at -18°C.

### EXAMPLES

### Materials and methods

Oxygen reduced sterile saline: 0.9% sodium chloride in demineralized water
DRcaps^{™}Capsules and Vcap^{®} capsules sizes 0 and 00 transparent or white (Capsugel)
Glycerol ("Farmaceutisk kvalitet" 99.5%, Vera Cura A/S)
Olive oil (Primadonna Bio Organic, Lidl)
Tuna oil (Omevital 0525 TG TUNA, BASF)
Rapeseed oil (VitaDór, Lidl)
Calcium silicate (372668 Aldrich, Sigma-Aldrich)
Potato starch (Coop^{®} Denmark)
Corn starch (Maizena^{®}, Unilever)
*L. fermentum* deposited as DSM32086
*Bifidobacterium longum* subsp. *infantis* BB-02 deposited as DSM15953

### EXAMPLE 1

### PREPARATION OF CAPSULES WITH FECAL MATTER

### Donor selection

Donors were selected using a questionnaire like the one used when recruiting blood donors. Based on 200 applicants, 20 applicants were selected for an interview and a second questionnaire. The second questionnaire was specifically looking for illnesses and diseases linked to disturbances in the microbiota of the applicant and his or her close relatives. These included obesity, IBS, IBD, neurological disorders, mental diseases, colorectal cancer, among others. 6 applicants were found qualified. Fecal samples from these 6 people were assessed for alfa-diversity using 16s rRNA gene amplicon sequencing. The 4 donors with the highest diversity were finally selected. Once recruited, the donors were instructed to keep up a healthy lifestyle during the collecting period.

All donors were screened vigorously for infectious disease and pathogens etc. before (first screening) and after the collecting period (second screening). Not until the donors had successfully passed the second screening their samples which had been kept as frozen fecal extracts at -18°C were used for the preparation of capsules with fecal matter.

### Preparation of feces extract

Donors were equipped with 500mL bottles of oxygen reduced sterile saline and instructed to immediately after producing a sample of feces to cover the sample with the oxygen reduced saline solution in order to protect the sample from oxygen and to bring it to the laboratory as soon as possible to ensure that no more than 3 hours later the sample could be processed in the laboratory.

To protect the sample against oxygen most of the processing in the laboratory was done in an anaerobic environment either by using an anaerobic chamber or by flushing with Ar. Feces and saline was homogenized, filtered and centrifuged. The supernatant was discarded, and the pellet was mixed with glycerol as a cryo-protectant to provide feces extract. Finally, the feces extract was frozen at -18°C.

### Pretest of size of cavity between the two capsules

45 µL, 90 µL, 110 µL, 130 µL, or 140 µL of oil, respectively, was added to a size 00 capsule before a smaller capsule (size 0) was inserted into the bigger capsule (size 00). In the capsules to which was added 45 µL and 90 µL of oil, respectively, the oil did not reach the opening of the capsule when the smaller capsule was inserted whereas the oil reached the opening of the bigger capsule when 130 µL oil was added to the bigger capsule before the smaller capsule was inserted. Adding 150 µL of oil was too large a volume (for these capsules) as the oil flew out of the bigger capsule when the smaller capsule was inserted into the bigger capsule. The conclusion was that for this combination of capsule sizes, 130 µL is the optimal amount of oil which should be added.

### Preparation of capsules with fecal extract

Size 0 capsules were filled with feces extract and as soon as possible the size 0 capsules were placed in size 00 capsules. The temperature of the feces extract was about -5°C so that no condensation was formed on the outside of the capsule. The capsule preparation was made by hand but could also be made by use of a capsule machine.

Trial 1 was made as a reference where nothing was added to the cavity between the two capsules.

Trial 2 was made to investigate if a hydrophilic liquid between the two capsules would improve the storage stability of the capsules. 130 µL of 99.5% glycerol was added to the cavity between the two capsules.

In trials 3 to 5 130 µL of olive oil, tuna fish oil or rapeseed oil, respectively, was added to the capsules. The theory was that by adding hydrophobic oil, water from the feces extract was prevented from migrating to the outer capsule or the rate of penetration of the inner capsule was slowed down dramatically.

In trials 6 and 7 2% of the desiccant calcium silicate was added to the feces extract. In trial 6 no oil was added to the cavity between the two capsules in order to test if a desiccant alone is as good as adding oil. In trial 7 both desiccant and oil were added.

In order to test if food approved desiccants have the same effect as silica desiccants, potato and corn starch were tested. In trial 8 10% potato starch was added to the feces extract and 130 µL of oil was added to the cavity between the two capsules.

In trial 9 the 10% potato starch was exchanged with 10% corn starch, otherwise exactly the same procedure as in trial 8.

### Results from trials with feces extract

**Table 2**

| Capsules with feces extract | Feces extract + additives | Ingredient between the capsules | Description of the capsules after storage at -18°C |
|---|---|---|---|
| Trial | | | |
| 1 | None | None | Sticky after few days, leakage after storage for 1-2 weeks |
| 2 | None | Glycerol (99.5%) (130 µL) | Sticky after 10 days - trial stopped |
| 3 | None | Olive oil (130 µL) | Sticky but no leakage after storage for 2½ months |
| 4 | None | Tuna fish oil (130 µL) | Sticky but no leakage after storage for at least 1½ months |
| 5 | None | Rapeseed oil (130 µL) | Sticky but no leakage after storage for 2½ months |
| 6 | 2% calcium silicate | None | Sticky after 1 month |
| 7 | 2% calcium silicate | Rapeseed oil (130 µL) | Neither stickiness nor leakage after storage for 3 months |
| 8 | 10% potato starch | Olive oil (130 µL) | Neither stickiness nor leakage after storage for more than 3 months |
| 9 | 10% corn starch | Olive oil (130 µL) | Neither stickiness nor leakage after storage for more than 3 months |

### Conclusion

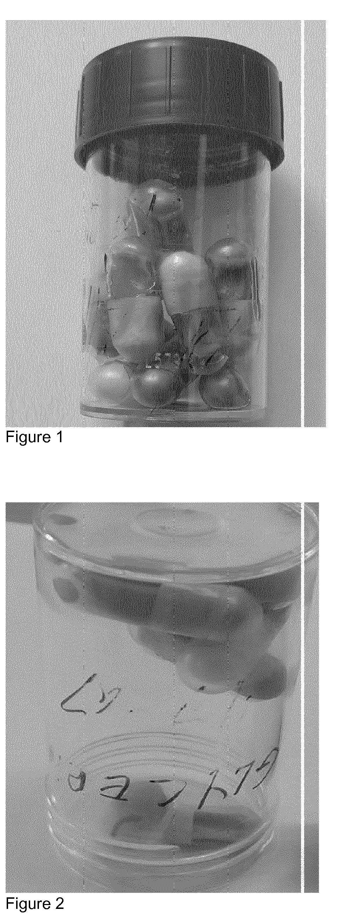

The capsules of trial 1 where nothing was added to the cavity were sticky and leaking after storage at -18°C for 1 week (Figure 1). The result is not surprising due to the fact that it is expected that water (liquid phase) from the feces extract will leak to the surface of the capsules leading to slow dissolution (swelling) of the capsule material making this permeable to water.

The capsules of trial 2 to which 130 µL of 99.5% glycerol has been added to the cavity were sticky after 10 days (Figure 2). This means that the stickiness appeared a little later than without any addition. A possible explanation is that water from the feces extract which has migrated through the inner capsule is diluted in the glycerol thereby prolonging the time until the stickiness appears. Even though the time before stickiness appeared was increased, the storage stability was still far from satisfactory.

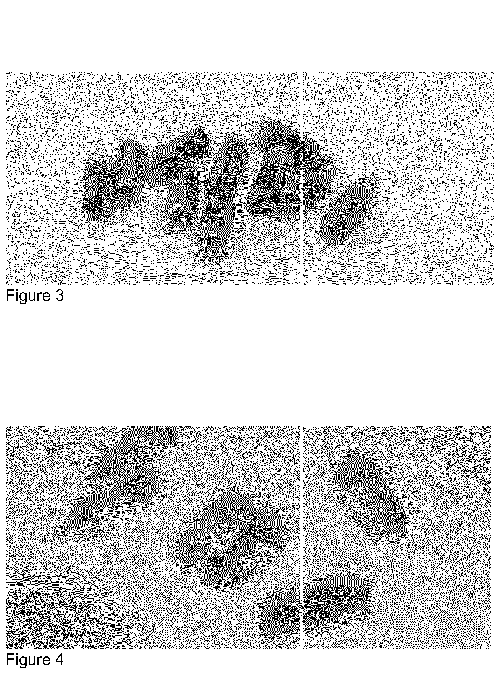

The capsules of trials 3 to 5 (Figures 3 and 4) had dramatically increased storage stability. After 2½ months the capsules of trials 3 to 5 were sticky but still not leaking.

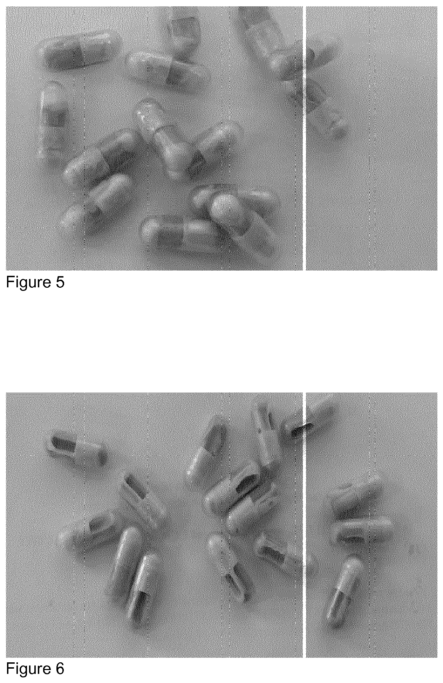

Adding 2% calcium silicate (trial 6) (Figure 5) improved the storage stability compared to the reference, trial 1, but not as much as adding oil, trials 3 to 5. In trial 7 where both 2% calcium silicate and oil were added, the storage stability was even better than when adding oil only (Figure 6). Use of aluminium silicate in lieu of calcium silicate was tested and gave almost same the results as for calcium silicate (data not shown).

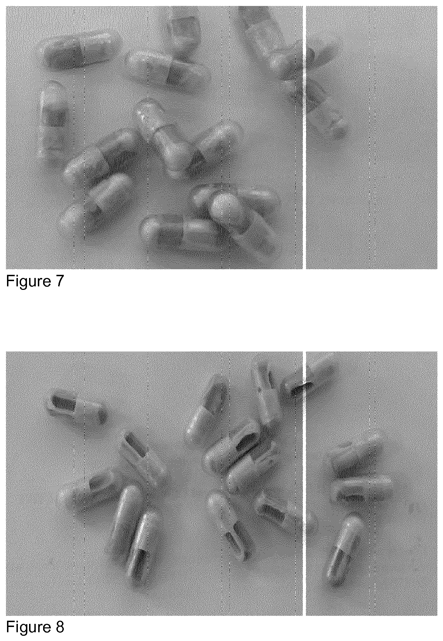

Capsules containing potato starch and oil (trial 8) (Figure 7) had very good storage stability when stored at -18°C and the same positive result as with potato starch was obtained with corn starch (trial 9) (Figure 8).

In summary, it is evident from the table and the figures that the storage stability of the capsules was improved dramatically by adding oil in the cavity between the two capsules. The hydrophobic oil prevents water from feces extract to reach the outer capsule thereby preventing the outer capsule from being sticky and possibly solubilizing.

By adding a desiccant to the feces extract and oil in the cavity between the two capsules even better storage stability was achieved.

### EXAMPLE 2

### PREPARATION OF A CAPSULE TEST SYSTEM

### Preparation of capsules with glycerol and water

In order to test the effect of adding various hydrophobic liquids, a model capsule system was developed comprising an outer and an inner capsule, wherein the outer capsule comprises a hydrophobic liquid and the inner capsule comprising water and glycerol in the inner capsule instead of microorganisms. The water content, app. 67%, of the composition in the inner capsule is much higher than will normally be found in a composition comprising microorganisms but can provide faster results.

Size 0 capsules were filled with a mixture of deionized water and glycerol (2:1) and as soon as possible the size 0 capsules were placed in size 00 capsules. To some of the size 00 capsules nothing was added, to other size 00 capsules 130 µL of rapeseed oil and olive oil, respectively, were added. The capsules were placed at -18°C and the stickiness was followed. The results are provided in Table 1.

### Results

**Table 1**

| Capsules with water and glycerol (2:1) | | |
|---|---|---|
| Trial | Capsules | Storage at -18°C |
| 10 | None | Sticky after 1 day |
| 11 | 130 µL rapeseed oil | Slightly sticky after 4 weeks |
| 12 | 130 µL olive oil | Sticky after 2½ weeks |

### Conclusion

The conclusion was that oil between the inner and outer capsules prolonged the time before the capsules became sticky.

### EXAMPLE 3

### PREPARATION OF CAPSULES WITH LACTIC ACID BACTERIA

### Preparation of capsules with L. fermentum

Glycerol was added to a concentrate of *L. fermentum* containing a dry matter of 15% in the proportion concentrate:glycerol 2:1. The mixture of culture and glycerol was filled in size 0 capsules and as soon as possible the size 0 capsules were placed in size 00 capsules. To some of the size 00 capsules nothing was added, to other capsules 130 µL of rapeseed oil or olive oil was added. The capsules were placed at -18°C and the stickiness was followed. The results are provided in Table 3.

### Results

**Table 3**

| Capsules with concentrate of *L. fermentum* and glycerol (2:1) | | |
|---|---|---|
| Trial | Capsules | Storage at -18°C |
| 13 | None | Sticky after 4 day |
| 14 | 130 µL rapeseed oil | Not sticky after 4 weeks |
| 15 | 130 µL olive oil | Not sticky after 4 weeks |

### Preparation of capsules with Bifidobacterium longum subsp. infantis BB-02 containing a dry matter of 12.9%

Glycerol was added to a concentrate of *Bifidobacterium longum* subsp. *infantis* BB-02 containing a dry matter of 12.9% in the proportion concentrate:glycerol 2:1. The mixture of concentrate and glycerol was filled in size 0 capsules and as soon as possible the size 0 capsules were placed in size 00 capsules. To some of the size 00 capsules nothing was added, to other capsules 130 µL of rapeseed oil or olive oil was added. The capsules were placed at -18°C and the stickiness was followed. The results are provided in Table 4.

### Results

**Table 4**

| Capsules with concentrate of *Bifidobacterium longum* subsp. *infantis* BB-02 (dry matter 12.9%) and glycerol (2:1) | | |
|---|---|---|
| Trial | Capsules | Storage at -18°C |
| 16 | None | Sticky after 4-6 days |
| 17 | 130 µL rapeseed oil | Not sticky after 3 weeks |
| 18 | 130 µL olive oil | Not sticky after 3 weeks |

After 11 months the capsules with oil were not leaking but were sticky and slightly free flowing, but not as much as for the capsules with a dry matter of 17.4% in the concentrate.

### Preparation of capsules with Bifidobacterium longum subsp. infantis BB-02 containing a dry matter of 17.4%

Concentrate of *Bifidobacterium longum* subsp. *infantis* BB-02 was concentrated by centrifugation to a dry matter of 17.4% before glycerol was added (2:1). The mixture of culture and glycerol was filled in size 0 capsules and as soon as possible the size 0 capsules were placed in size 00 capsules. To some of the size 00 capsules nothing was added, to other capsules 130 µL of rapeseed oil or olive oil was added. The capsules were placed at -18°C and the stickiness was followed. The results are provided in Table 5.

### Results

**Table 5**

| Capsules with concentrate of *Bifidobacterium longum* subsp. *infantis* BB-02 (dry matter 17.4%) and glycerol (2:1) | | |
|---|---|---|
| Trial | Capsules | Storage at -18°C |
| 19 | None | Sticky after 4-6 days |
| 20 | 130 µL rapeseed oil | Not sticky after 3 weeks |
| 21 | 130 µL olive oil | Not sticky after 3 weeks |

After 11 months the capsules with oil were not leaking but were sticky and slightly free flowing, i.e. the capsules were less sticky than the capsules with a dry matter of 12.9% in the concentrate.

### DEPOSITS AND EXPERT SOLUTION

*Bifidobacterium longum* subsp. *infantis* BB-02 deposited as DSM 15953 is referred to in European patent EP 2318511.

The applicant requests that a sample of the deposited micro-organism stated below may only be made available to an expert, until the date on which the patent is granted.

The *Lactobacillus fermentum* strain DSM 32086 was deposited at Deutsche Sammlung von Mikroorganismen und Zellkulturen GmbH; DSMZ, Inhoffenstr. 7B, D-38124 Braunschweig on July 16, 2015.

## Claims

1. A hard capsule comprising a hard outer and one inner hard capsule, wherein the outer capsule comprises an edible oil and the inner hard capsule which comprises a composition comprising microorganisms which are not coated or microencapsulated and at least one desiccant, wherein the hard capsule has a load of microorganisms of at least 30% weight/volume.

2. A capsule according to claim 1, wherein the composition comprises at least one bacterium, archaea, fungus, or virus.

3. A capsule according to claim 1 or 2, wherein the composition comprises fecal microbiota.

4. A capsule according to claim 1 or 2, wherein the composition comprises no more than 20 different types of microorganisms.

5. A capsule according to claim 4, wherein the composition comprises no more than 20 different types of bacteria.

6. A capsule according to any of claims 1 to 5, wherein the at least one edible oil is sunflower oil, olive oil, rapeseed oil, maize oil, soya oil, linseed oil, groundnut oil, sesame oil, rice oil, thistle oil, grape seed oil, peanut oil, coconut oil, and fish oil.

7. A capsule according to any one of claims 1 to 6, wherein the edible oil comprises at least one antioxidant.

8. A capsule according to claim 7, wherein the at least one antioxidant is ascorbyl palmitate (E304), tocopherol- rich extract (E306), alpha-tocopherol (E307), gamma-tocopherol (E308), delta-tocopherol (E309), propyl gallate (E310), octyl gallate (E311), dodecyl gallate (E312), β-carotenoids, tertiary-butyl hydroxyquinone (TBHA) (E319), butylated hydroxyanisole (BHA) (E320), butylated hydroxytolvene (BHT) (E321), lecithin (E322), 4-hexylresorcinol (E586), or combinations thereof.

9. A capsule according to any one of claims 1-8, wherein the desiccant is a food approved component which is degradable in the gastrointestinal tract.

10. A capsule according to any one of claims 1 to 9, wherein the desiccant is starch, such as potato starch, corn starch, rice starch, wheat starch, or cassava starch.

11. A capsule according to any one of claims 1 to 10, wherein the composition comprises at least one cryoprotectant.

12. A capsule according to claim 11, wherein the cryoprotectant is glycerol, carbohydrate, water soluble antioxidants as e.g. sodium ascorbate, glutathione, riboflavin, L-cysteine, or salts or combinations thereof.

13. A capsule according to any one of claims 1 to 12 for the use in the prevention or treatment of an infection or a disease, disorder or condition associated with a dysbiosis.

14. A capsule according to any one of claims 1 to 12 for the use in preventing or treating an infection caused by C. *difficile, Salmonella* spp., enteropathogenic *E coli,* multi-drug resistant bacteria such as *Klebsiella,* and *E. coli,* Carbapenem-resistent *Enterobacteriaceae* (CRE), extended spectrum beta-lactam resistant *Enterococci* (ESBL), and vancomycin-resistant *Enterococci* (VRE).

## Patentansprüche

1. Harte Kapsel, umfassend eine harte Außenkapsel und eine innere harte Kapsel, wobei die Außenkapsel ein Speiseöl umfasst und die innere harte Kapsel eine Zusammensetzung umfasst, die Mikroorganismen, die nicht beschichtet oder mikroverkapselt sind, und mindestens ein Trockenmittel umfasst, wobei die harte Kapsel eine Beladung an Mikroorganismen von mindestens 30 % Gewicht/Volumen aufweist.

2. Kapsel nach Anspruch 1, wobei die Zusammensetzung mindestens ein Bakterium, Archaeon, einen Pilz oder ein Virus umfasst.

3. Kapsel nach Anspruch 1 oder 2, wobei die Zusammensetzung fäkale Mikrobiota umfasst.

4. Kapsel nach Anspruch 1 oder 2, wobei die Zusammensetzung nicht mehr als 20 verschiedene Arten von Mikroorganismen umfasst.

5. Kapsel nach Anspruch 4, wobei die Zusammensetzung nicht mehr als 20 verschiedene Arten von Bakterien umfasst.

6. Kapsel nach einem der Ansprüche 1 bis 5, wobei das mindestens eine Speiseöl Sonnenblumenöl, Olivenöl, Rapsöl, Maisöl, Sojaöl, Leinöl, Erdnussöl, Sesamöl, Reisöl, Distelöl, Traubenkernöl, Erdnussöl, Kokosöl und Fischöl ist.

7. Kapsel nach einem der Ansprüche 1 bis 6, wobei das Speiseöl mindestens ein Antioxidans umfasst.

8. Kapsel nach Anspruch 7, wobei das mindestens eine Antioxidans Ascorbylpalmitat (E304), tocopherolreicher Extrakt (E306), Alpha-Tocopherol (E307), Gamma-Tocopherol (E308), Delta-Tocopherol (E309), Propylgallat (E310), Octylgallat (E311), Dodecylgallat (E312), β-Carotinoide, Tertiärbutylhydrochinon (TBHA) (E319), butyliertes Hydroxyanisol (BHA - butylated hydroxyanisole) (E320), butyliertes Hydroxytoluol (BHT - butylated hydroxytolvene) (E321), Lecithin (E322), 4-Hexylresorcinol (E586) oder Kombinationen davon ist.

9. Kapsel nach einem der Ansprüche 1-8, wobei das Trockenmittel ein lebensmittelzugelassener Bestandteil ist, der im Gastrointestinaltrakt abbaubar ist.

10. Kapsel nach einem der Ansprüche 1 bis 9, wobei das Trockenmittel Stärke ist, wie Kartoffelstärke, Maisstärke, Reisstärke, Weizenstärke oder Kassavastärke.

11. Kapsel nach einem der Ansprüche 1 bis 10, wobei die Zusammensetzung mindestens einen Kryoprotektant umfasst.

12. Kapsel nach Anspruch 11, wobei der Kryoprotektant Glycerol, Kohlenhydrat, wasserlösliche Antioxidantien wie z. B. Natriumascorbat, Glutathion, Riboflavin, L-Cystein oder Salze oder Kombinationen davon ist.

13. Kapsel nach einem der Ansprüche 1 bis 12 zur Verwendung bei der Vorbeugung oder Behandlung einer Infektion oder einer Krankheit, Störung oder eines Zustands, der mit einer Dysbiose assoziiert ist.

14. Kapsel nach einem der Ansprüche 1 bis 12 zur Verwendung beim Vorbeugen oder Behandeln einer Infektion, die durch C. *difficile, Salmonella* spp., enteropathogene *E coli,* multiresistente Bakterien wie *Klebsiella* und *E. coli,* Carbapenem-resistent *Enterobacteriaceae* (CRE), extendedspectrum beta-lactam-resistente *Enterokokken* (ESBL) und Vancomycin-resistente *Enterokokken* (VRE) verursacht wird.

## Revendications

1. Capsule dure comprenant une capsule externe dure et une capsule interne dure, dans laquelle la capsule externe comprend une huile comestible et la capsule interne dure qui comprend une composition comprenant des micro-organismes qui ne sont ni enrobés ni microencapsulés et au moins un dessiccant, dans laquelle la capsule dure a une charge de micro-organismes d'au moins 30 % en poids/volume.

2. Capsule selon la revendication 1, dans laquelle la composition comprend au moins une bactérie, une archée, un champignon ou un virus.

3. Capsule selon la revendication 1 ou 2, dans laquelle la composition comprend du microbiote fécal.

4. Capsule selon la revendication 1 ou 2, dans laquelle la composition comprend au plus 20 types différents de micro-organismes.

5. Capsule selon la revendication 4, dans laquelle la composition comprend au plus 20 types différents de bactéries.

6. Capsule selon l'une quelconque des revendications 1 à 5, dans laquelle l'au moins une huile comestible est de l'huile de tournesol, de l'huile d'olive, de l'huile de colza, de l'huile de maïs, de l'huile de soja, de l'huile de lin, de l'huile d'arachide, de l'huile de sésame, de l'huile de riz, de l'huile de chardon, de l'huile de pépins de raisin, de l'huile d'arachide, de l'huile de noix de coco et de l'huile de poisson.

7. Capsule selon l'une quelconque des revendications 1 à 6, dans laquelle l'huile comestible comprend au moins un antioxydant.

8. Capsule selon la revendication 7, dans laquelle l'au moins un antioxydant est le palmitate d'ascorbyle (E304), un extrait riche en tocophérol (E306), l'alpha-tocophérol (E307), le gamma-tocophérol (E308), le delta-tocophérol (E309), le gallate de propyle (E310), le gallate d'octyle (E311), le gallate de dodécyle (E312), les β-caroténoïdes, la tert-butylhydroxyquinone (TBHA) (E319), l'hydroxyanisole butylé (BHA) (E320), l'hydroxytoluène butylé (BHT) (E321), la lécithine (E322), le 4-hexylrésorcinol (E586), ou des combinaisons de ceux-ci.

9. Capsule selon l'une quelconque des revendications 1 à 8, dans laquelle le dessiccant est un composant alimentaire approuvé qui est dégradable dans le tractus gastro-intestinal.

10. Capsule selon l'une quelconque des revendications 1 à 9, dans laquelle le dessiccant est de l'amidon, tel que de l'amidon de pomme de terre, de l'amidon de maïs, de l'amidon de riz, de l'amidon de blé ou de l'amidon de manioc.

11. Capsule selon l'une quelconque des revendications 1 à 10, dans laquelle la composition comprend au moins un cryoprotecteur.

12. Capsule selon la revendication 11, dans laquelle le cryoprotecteur est du glycérol, des glucides, des antioxydants hydrosolubles tels que par exemple l'ascorbate de sodium, le glutathion, la riboflavine, la L-cystéine, ou des sels ou des combinaisons de ceux-ci.

13. Capsule selon l'une quelconque des revendications 1 à 12 pour l'utilisation dans la prévention ou le traitement d'une infection ou d'une maladie, d'un trouble ou d'une affection associée à une dysbiose.

14. Capsule selon l'une quelconque des revendications 1 à 12 pour l'utilisation dans la prévention ou le traitement d'une infection causée par C. *difficile, Salmonella* spp., *E.* colientéropathogène, des bactéries multirésistantes telles que *Klebsiella* et *E. coli, des entérobactéries* résistantes aux carbapénèmes (CRE), *des entérocoques* résistants aux bêta-lactamines à spectre étendu (ESBL) et *des entérocoques* résistants à la vancomycine (VRE).
